# DEMANDE DE BREVET EUROPEEN

(11) **EP 2 503 150 A1**
(43) Date de publication de la demande: **26.09.2012**
(21) Numéro de dépôt: 11159081.6
(22) Date de dépôt: 21.03.2011
(51) Int. Cl.: F04B 43/12, F04B 49/06, A61M 5/168, A61M 5/142

(54) **Dispositif pour la détection et la mesure de la rotation d'une cassette péristaltique**

(71) Demandeur: SMC-Swiss Medical Care S.A., 1004 Lausanne (CH)
(72) Inventeur: Neftel, Frédéric, 1005 Lausanne (CH)
(74) Mandataire: Grosfillier, Philippe

(57) **Abrégé**

Dispositif pour la détection et l'identification d'une cassette péristaltique dotée d'un mécanisme rotatif comprenant des galets liés à un spacer (1) et destinée à être entrainée en rotation par un système d'entrainement lié au dispositif. Ledit dispositif comprend un capteur disposé de manière à détecter le passage d'au moins un élément distinctif (2) disposé sur le dit spacer, par comptage du nombre d'éléments distinctifs détectés au cours d'une rotation de la cassette.

## Description

### Domaine de l'invention

La présente invention concerne les cassettes péristaltiques qui comprennent un ensemble de galets entraînés par rotation autour d'un axe. L'invention est particulièrement destinée au domaine médical et vise à assurer une reconnaissance d'une telle cassette par le système d'entrainement afin d'éviter l'usage de contre façons, des problèmes de fonctionnement et/ou des caractéristiques propres à la cassette utilisée.

### Etat de la technique

Un exemple de cassette correspondant à la définition du chapitre précédent est divulgué dans la demande de brevet WO 2005/106251, ainsi que dans le brevet Malbec EP0388269.

La cassette comporte un ensemble de galets effectuant un mouvement de rotation autour d'un axe moteur central, chaque galet possède un axe dont au moins une extrémité est montée coulissante, selon une direction radiale, dans la fente d'une plaque nommée « spacer » disposée sur une face de la cassette, cette plaque étant elle-même soit rendue solidaire de l'axe moteur, soit entrainée par la rotation des galets, eux même entrainés par exemple par friction avec l'axe central.

La vitesse de rotation des galets doit être déterminée avec la plus grande précision possible car elle permet de déterminer la vitesse d'écoulement ou le débit du liquide qui se déplace à travers la cassette.

### Exposé général de l'invention

Le problème que la présente invention se propose de résoudre réside dans une amélioration de la détection et de la mesure de la rotation d'une cassette péristaltique et/ou de l'identification d'une cassette.

Dans l'invention, la solution du problème précité consiste en un dispositif pour la détection et l'identification d'une cassette péristaltique dotée d'un mécanisme rotatif comprenant des galets liés à un spacer et destinée à être entrainée en rotation par un système d'entrainement lié au dispositif ; ledit dispositif étant caractérisé par le fait qu'il comprend un capteur solidaire du système d'entrainement disposé de manière à détecter le passage d'au moins un élément distinctif disposé sur le dit spacer par comptage du nombre d'éléments distinctifs détectés au cours d'une rotation de la cassette.

Le spacer est en principe défini essentiellement par deux face opposées, soit une face interne dirigée vers l'intérieur de la cassette et une face externe dirigée vers l'extérieur de la cassette. Le dispositif est dans ce cas caractérisé par le fait qu'il comprend un capteur disposé de manière à détecter le passage d'au moins un élément distinctif visible sur ladite face externe. Le spacer peut également être lié aux galets, apr tout autre moyen, afin d'assurer une rotation qui soit proportionnelle à la rotation des galets.

De préférence, les éléments distinctifs sont situés sur une couronne venant en regard dudit capteur pendant la rotation de la cassette. Ces éléments distinctifs n'ont pas nécessairement d'autre fonction et se surajoutent dans ce cas aux éléments fonctionnels de la cassette dans le but unique d'assurer cette détection ou cette identification de la cassette.

Dans un mode de réalisation de l'invention le capteur est optique. Dans ce cas le spacer est soit doté d'orifices pouvant être détectés par le capteur optique au cours de la rotation du spacer, soit de régions optiquement réfléchissantes ou opaques (surfaces marquées) permettant d'assurer une détection de ces surfaces au moment de leur passage en regard du capteur et ainsi une identification de la cassette.

Les orifices ou surfaces marquées peuvent être réparties de façon symétrique ou asymétrique sur un diamètre du spacer situé en regard du capteur optique et ainsi représenter une information codée permettant notamment d'identifier le type de cassette utilisée. Afin de pouvoir lire l'information ainsi portée par le spacer, il est nécessaire d'effectuer au moins un tour complet de cassette.

Une identification de ce type peut être particulièrement utile lorsqu'il s'agit d'éviter des contrefaçons de cassette destinées à un usage médical afin d'assurer que seules les cassettes garanties par le fabriquant original puissent être utilisées par le dispositif d'entrainement.

Un exemple est celui des injecteurs de produits de contrastes, nécessitant une grande précision de fonctionnement permettant d'assurer que l'injection au patient n'est pas interrompue ou altérée. Un défaut de fonctionnement en cours d'injection représenterait un risque important d'avoir à répéter l'examen du Scanner CT et donc un risque d'irradier le patient une deuxième fois avec une dose parfois importante de Rayons X. En utilisant un tel mécanisme permettant d'identifier une cassette originale (par exemple du fait de la nature de la séquence des orifices ou des surfaces marquées reconnues au cours d'une rotation de cassette) on empêche l'utilisation de cassette ne provenant pas du fabriquant original. Un tel marquage de cassette se situe à l'intérieur de la cassette et nécessite un fonctionnement de la cassette qui doit être mise en rotation dans l'appareil (par exemple l'injecteur) pour être détectée. L'utilisateur ne peut ainsi fausser ce mode de détection par l'ajout, par exemple, d'un marquage ou d'une étiquette à l'extérieur de la cassette, ni par une ouverture de la cassette qui doit rester stérile et qui serait éventuellement détruite au cours d'une telle opération. En outre, la détection de la rotation de la cassette durant l'injection permet de s'assurer de son bon fonctionnement et de la quantité réellement injectée au cours du temps par comptage du nombre de rotation effectuée par la cassette et en la comparant avec le nombre de rotation attendue du fait du nombre de rotation effective de l'axe d'entrainement.

Un tel système de marquage de la cassette permet également d'informer l'injecteur du type de cassette utilisée, de ses performances et/ou de sa date de péremption, ceci afin d'éviter l'usage d'une cassette périmée. La séquence des marquages et leur écartement permet ainsi de porter l'information spécifique souhaitée.

Un exemple d'utilisation de l'invention dans le cadre des injecteurs de produits de contraste pour imagerie par Scanner CT, tels que le CT Expres 3D et 4D commercialisés par le déposant, est la reconnaissance et la distinction entre des cassettes destinées à un patient unique (CT Expres 3D) et des cassettes permettant un usage pour de multiples patients (CT Expres 4D), cette dernière étant prévue pour un usage plus long pouvant atteindre 4 litres injectés contre 300ml pour les autres cassettes.

Il va de soi que l'invention ne se limite pas aux seuls exemples de réalisation décrits ci-dessus et peut être avantageusement utilisées dans tout type de dispositif nécessitant une identification d'un type de système rotatif pour l'administration de fluide, tout en assurant une fiabilité élevée contre toute contrefaçon.

### Exposé détaillé de l'invention

L'invention est décrite plus en détail ci-après au moyen d'exemples illustrés par les figures suivantes :
La figure 1 illustre divers coupes et vues d'un spacer selon l'invention
La figure 2 montre une capture d'écran d'oscilloscope qui affiche le signal enregistré par un capteur optique.

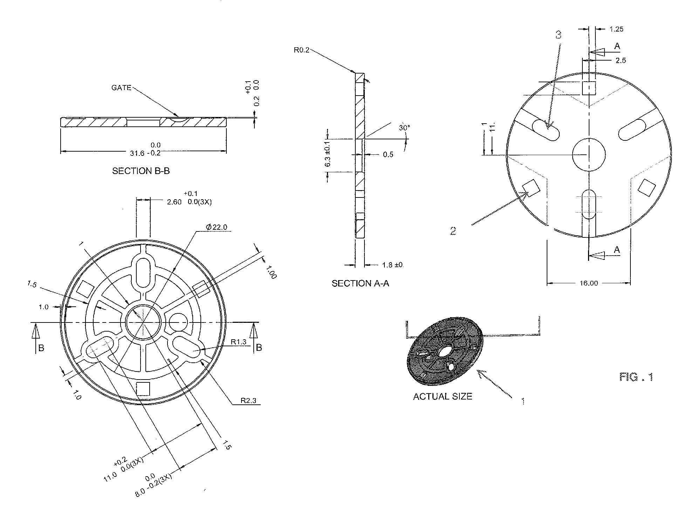

La figure 1 représente un spacer **1** de forme assez similaire à celui illustré dans la demande WO 2005/106251 mais qui diffère en ce qu'il comporte trois orifices **2** disposés sur une couronne dont le diamètre est supérieure à une autre couronne dans laquelle sont disposées les fentes **3** de guidage des galets.

Dans cet exemple, les trois orifices **2** ont des sections carrées. N'importe quelle autre forme de section ou nombre d'orifices peuvent être utilisés.

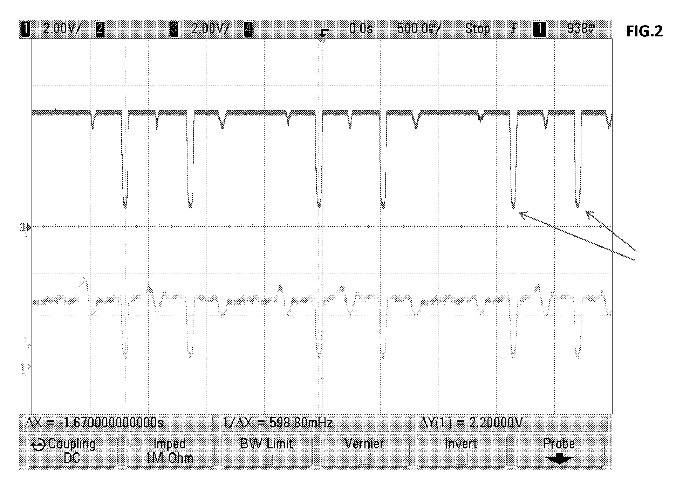

La courbe d'oscilloscope de la figure 2 montre des vallées (illustrées par des flèches) qui révèlent le passage des orifices devant le capteur optique. La forme générale de cette courbe permet donc de déterminer aisément et rapidement le type de cassette (car chaque type possède un nombre spécifique d'orifices) ainsi que son authenticité.

## Revendications

1. Dispositif pour la détection et l'identification d'une cassette péristaltique dotée d'un mécanisme rotatif comprenant des galets liés à un spacer et destinée à être entrainée en rotation par un système d'entrainement lié au dispositif ; ledit dispositif étant **caractérisé par le fait qu'**il comprend un capteur disposé de manière à détecter le passage d'au moins un élément distinctif disposé sur le dit spacer, par comptage du nombre d'éléments distinctifs détectés au cours d'une rotation de la cassette.

2. Dispositif selon les revendications 1 dans lequel le spacer est défini essentiellement par deux face opposées, soit une face interne dirigée vers l'intérieur de la cassette et une face externe dirigée vers l'extérieur de la cassette, l'élément distinctif étant au moins détectable sur ladite face externe.

3. Dispositif selon la revendication 1 à 2 dans lequel le capteur est un capteur optique.

4. Dispositif selon la revendication 1 à 2 dans lequel le capteur est un capteur a effet hall et l'élément distinctif un aimant.

5. Cassette destinée à être utilisée avec un dispositif selon l'une des revendications 1 à 3 dans laquelle l'élément distinctif est une surface réfléchissante.

6. Cassette destinée à être utilisée avec un dispositif selon l'une des revendications 1 à 3 dans laquelle l'élément distinctif est un orifice.

7. Cassette selon la revendication 6 comprenant plusieurs éléments distinctifs en forme d'orifices.

8. Cassette selon la revendication 5 ou 7 dans laquelle au moins un orifice ou une surface réfléchissante comporte une longueur différente des autres orifices dans l'axe de lecture en rotation par le capteur optique.

9. Cassette selon la revendication 8 dans laquelle les orifices ou surfaces réfléchissantes sont disposés à intervalles irréguliers.

10. Cassette selon l'une des revendications 8 à 10 dans laquelle les intervalles représentent une séquence d'identification spécifique de la cassette.

11. Cassette selon l'une des revendications 6 à 10 dans laquelle le ou les éléments distinctifs sont disposés sur une trajectoire circulaire du spacer.

12. Ensemble constitué d'un dispositif et d'une cassette tels que défini dans l'une quelconque des revendications précédentes.

13. Méthode de détermination de la vitesse de rotation d'une cassette telle que définie dans les revendications précédentes comprenant une étape dans laquelle on calcule le nombre d'éléments distinctifs détectés par un capteur au cours d'un intervalle de temps prédéfini, la dite vitesse étant comparée avec la vitesse attendue en fonction de la vitesse d'entrainement de l'axe de la cassette.

14. Méthode de différenciation de cassettes péristaltiques à usage unique et à usage multiple, les cassettes étant telles que définies dans les revendications précédentes, les deux types de cassettes se différenciant par le nombre d'éléments distinctifs disposés sur la face externe du spacer ; ladite méthode consistant à différencier les deux types de cassettes par comptage du nombre d'éléments distinctifs détectés au cours d'une rotation au moins complète de la cassette.

15. Méthode de différenciation de cassettes péristaltiques à usage unique et à usage multiple selon la revendication 14 dans laquelle la cassette est entrainée à faible vitesse au moment de sa première insertion dans le système d'entrainement afin de lire l'information portée par le spacer au travers du ou des éléments distinctifs par comptage du nombre d'éléments distinctifs détectés au cours d'une rotation au moins complète de la cassette et ainsi déterminer son type préalablement à toute programmation d'injection de fluide.

16. Méthode de reconnaissance d'une cassette péristaltique originale dans laquelle la cassette est entrainée à faible vitesse au moment de sa première insertion dans le système d'entrainement afin de lire l'information portée par le spacer au travers du ou des éléments distinctifs par comptage du nombre d'éléments distinctifs détectés au cours d'une rotation au moins complète de la cassette et de déterminer son authenticité par comparaison avec une séquence de référence préalablement à toute programmation d'injection de fluide.

17. Utilisation d'un dispositif de détection tel que défini dans les revendications 1 à 4 pour assurer le bon fonctionnement d'une cassette péristaltique au cours de l'injection d'un fluide.

18. Utilisation d'un dispositif de détection tel que défini dans les revendications 1 à 4 pour la détection d'une cassette péristaltique, ladite utilisation comprenant une étape lors de laquelle on effectue une mesure pendant au moins une rotation complète de la cassette.
